# EUROPÄISCHE PATENTSCHRIFT

(11) **EP 2 797 666 B1**
(45) Veröffentlichungstag und Bekanntmachung des Hinweises auf die Patenterteilung: **19.07.2017**
(21) Anmeldenummer: 13703067.2
(22) Anmeldetag: 07.02.2013
(51) Int. Cl.: A61N 1/36, A61N 1/05

(54) **VORRICHTUNG ZUR EICHUNG EINER INVASIVEN, ELEKTRISCHEN UND DESYNCHRONISIERENDEN NEUROSTIMULATION**
APPARATUS FOR CALIBRATING INVASIVE ELECTRIC DESYNCHRONIZING NEUROSTIMULATION
DISPOSITIF D'ÉTALONNAGE D'UNE NEUROSTIMULATION ÉLECTRIQUE DÉSYNCHRONISANTE EFFRACTIVE

(30) Priorität: 08.02.2012 DE 102012002437
(43) Veröffentlichungstag der Anmeldung: 05.11.2014
(73) Patentinhaber: Forschungszentrum Jülich GmbH, 52425 Jülich (DE)
(72) Erfinder: TASS, Peter Alexander, 83684 Tegernsee (DE)
(74) Vertreter: Manitz Finsterwald Patentanwälte PartmbB
(86) Internationale Anmeldenummer: PCT/EP2013/052453
(87) Internationale Veröffentlichungsnummer: WO 2013/117656

(56) Entgegenhaltungen:
- WO-A1-03/043690
- WO-A2-2008/109508
- WO-A2-2011/127917
- US-B1- 6 597 954

## Beschreibung

Die Erfindung betrifft eine Vorrichtung zur Eichung einer invasiven, elektrischen und desynchronisierenden Neurostimulation.

Bei Patienten mit neurologischen oder psychiatrischen Erkrankungen, z.B. Morbus Parkinson, essentiellem Tremor, Dystonie oder Zwangserkrankungen, sind Nervenzellverbände in umschriebenen Bereichen des Gehirns krankhaft, z.B. übersteigert synchron, aktiv. In diesem Fall bildet eine große Anzahl von Neuronen synchron Aktionspotentiale aus, d.h., die beteiligten Neuronen feuern übermäßig synchron. Beim Gesunden hingegen feuern die Neuronen in diesen Hirngebieten qualitativ anders, z.B. auf unkorrelierte Weise.

Zur Behandlung derartiger Erkrankungen wurden Stimulationstechniken entwickelt, die gezielt krankhaft synchroner neuronaler Aktivität entgegenwirken. Insbesondere die "Coordinated Reset" (CR)-Stimulation zeichnet sich hierbei durch große therapeutische Wirksamkeit und Sicherheit aus (vgl. z.B. "A model of desynchronizing deep brain stimulation with a demand-controlled coordinated reset of neural subpopulations" von P. A. Tass, erschienen in Biol. Cybern. 89, 2003, Seiten 81 bis 88). Um gezielt in umschriebenen Zielgebieten des Gehirns eine desynchronisierende Wirkung zu entfalten, werden Elektroden (z.B. Tiefenelektroden) in diesen Zielgebieten und/oder in damit verbundenen Fasergebieten implantiert. Von zentraler Bedeutung für die Wirksamkeit der CR-Stimulation ist, dass die verschiedenen Stimulationskontakte in der zu stimulierenden Neuronenpopulation und/oder in damit verbundenen Fasergebieten liegen. Optimale CR-Stimulation kann nur mit mindestens zwei, besser mehr (z.B. vier und mehr) Stimulationskontakten durchgeführt werden. Insbesondere sind die optimalen Stimulationskontakte gemäß ihrer tatsächlichen Stimulationswirkung auf die zu desynchronisierende Neuronenpopulation auszuwählen. Zur Auswahl der N optimalen Stimulationskontakte aus einer größeren Anzahl M (mit M > N) an Stimulationskontakten gibt es bis jetzt kein automatisch funktionierendes Verfahren, welches objektive Messgrößen verwendet. Vielmehr erfolgt die Auswahl geeigneter Stimulationskontakte und der zugehörigen Parameter (z.B. Stimulationsamplitude) durch zeitaufwändiges Probieren. Dieser "trial and error"-Prozess garantiert die optimale Wirksamkeit der invasiven CR-Therapie nicht, da einerseits (insbesondere bei einer größeren Anzahl von Stimulationskontakten) nicht alle möglichen Stimulationsorte im Gehirn systematisch untersucht werden, und andererseits die Patienten durch lange Untersuchungen strapaziert werden, so dass die Mitarbeit der Patienten naturgemäß leidet, und die Ergebnisse der Testung schlechter werden.

Das Dokument WO 2011 / 127 917 A2 offenbart eine herkömmliche Vorrichtung zur Stimulation von Neuronen mit einer krankhaft synchronen und oszillatorischen neuronalen Aktivität. Die Vorrichtung umfasst eine Stimulationseinheit mit einer Mehrzahl von Stimulationskontakten zur Stimulation von Neuronen im Gehirn und/oder Rückenmark eines Patienten mit elektrischen Reizen, eine Messeinheit zum Aufnehmen von Messsignalen, die eine neuronale Aktivität der stimulierten Neuronen wiedergeben, und eine Steuer- und Analyseeinheit zur Steuerung der Stimulationseinheit und zur Analyse der Messsignale.

Der Erfindung liegt die Aufgabe zugrunde, eine Vorrichtung anzugeben, die eine vom Untersucher unabhängige, automatisch durchgeführte, elektrophysiologisch basierte Eichung der Stimulationsparameter ermöglicht. Insbesondere soll diese Eichung ermöglichen, (i) die Therapie wirksam durchzuführen, (ii) Nebenwirkungen zu vermeiden und (iii) die zur Parametereinstellung durchzuführende Untersuchung möglichst kurz, praktikabel und für den Patienten erträglich zu gestalten.

Die der Erfindung zugrunde liegende Aufgabenstellung wird durch die Merkmale des Anspruchs 1 gelöst. Vorteilhafte Weiterbildungen und Ausgestaltungen der Erfindung sind in den Unteransprüchen angegeben.

Die Erfindung wird nachfolgend in beispielhafter Weise unter Bezugnahme auf die Zeichnungen näher erläutert. In diesen zeigen:
- Fig. 1: eine schematische Darstellung einer Vorrichtung zur invasiven, elektrischen und desynchronisierenden Neurostimulation während des Betriebs;
- Fig. 2: ein Flussdiagramm zur Veranschaulichung der Eichung der in Fig. 1 gezeigten Vorrichtung gemäß einer ersten Variante;
- Fig. 3: eine schematische Darstellung einer Reizfolge zur Analyse der durch die Reize bewirkten Phasenrücksetzung;
- Fig. 4: eine schematische Darstellung einer CR-Stimulation mit zwei benachbarten Stimulationskontakten;
- Fig. 5: eine schematische Darstellung einer CR-Stimulation mit vier Stimulationskontakten;
- Fig. 6: eine schematische Darstellung eines zur Stimulation verwendeten elektrischen Pulszugs;
- Fig. 7: ein Flussdiagramm zur Veranschaulichung der Eichung der in Fig. 1 gezeigten Vorrichtung gemäß einer zweiten Variante;
- Fig. 8: ein Flussdiagramm zur Veranschaulichung der Eichung für eine Elektrode mit zirkulären Stimulationskontakten;
- Fig. 9: ein Flussdiagramm zur Veranschaulichung der Eichung für eine Elektrode mit kreisförmigen Stimulationskontakten;
- Fig. 10: eine schematische Darstellung einer Variante der CR-Stimulation;
- Fig. 11: eine schematische Darstellung einer weiteren Variante der CR-Stimulation; und
- Fig. 12: eine schematische Darstellung einer weiteren Vorrichtung zur EEG-basierten Eichung einer CR-Stimulation.

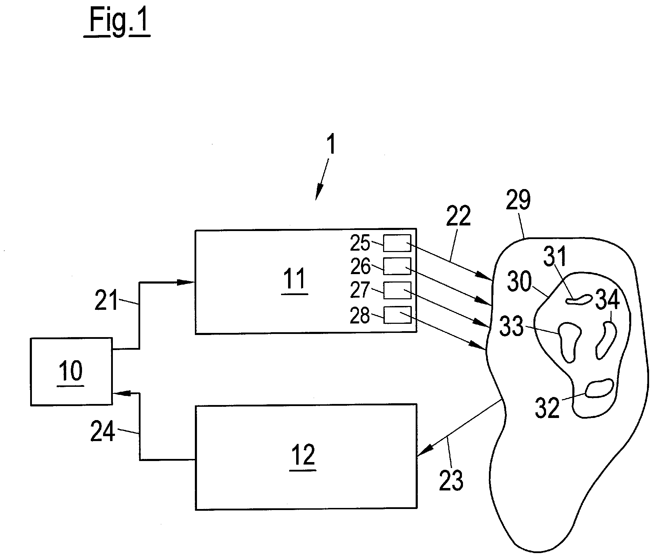

In Fig. 1 ist schematisch eine Vorrichtung 1 zur Eichung der Stimulationsparameter einer invasiven, elektrischen und desynchronisierenden Neurostimulation dargestellt. Die Vorrichtung 1 besteht aus einer Steuer- und Analyseeinheit 10, einer Stimulationseinheit 11 und einer Messeinheit 12. Während des Betriebs der Vorrichtung 1 führt die Steuer- und Analyseeinheit 10 u.a. eine Steuerung der Stimulationseinheit 11 durch. Dazu erzeugt die Steuer- und Analyseeinheit 10 Steuersignale 21, die von der Stimulationseinheit 11 entgegengenommen werden. Die Stimulationseinheit 11 wird operativ in den Körper des Patienten implantiert und erzeugt anhand der Steuersignale 21 elektrische Reize 22, die dem Gehirn und/oder Rückenmark des Patienten verabreicht werden. Die Steuer- und Analyseeinheit 10 und/oder die Messeinheit 12 können nicht-invasive Einheiten sein, d.h., während des Betriebs der Vorrichtung 1 befinden sie sich außerhalb des Körpers des Patienten und werden nicht operativ in den Körper des Patienten implantiert.

Der durch die elektrischen Reize 22 erzielte Stimulationseffekt wird mit Hilfe der Messeinheit 12 kontrolliert. Die Messeinheit 12 nimmt ein oder mehrere am Patienten gemessene Messsignale 23 auf, wandelt diese gegebenenfalls in elektrische Signale 24 um und führt diese der Steuer- und Analyseeinheit 10 zu. Insbesondere kann mittels der Messeinheit 12 die neuronale Aktivität in dem stimulierten Zielgebiet oder einem mit dem Zielgebiet verbundenen Gebiet gemessen werden, wobei die neuronale Aktivität dieses Gebiets mit der neuronalen Aktivität des Zielgebiets hinreichend eng korreliert. Die Steuer- und Analyseeinheit 10 verarbeitet die Signale 24, z.B. können die Signale 24 verstärkt und/oder gefiltert werden, und analysiert die verarbeiteten Signale 24. Anhand der Ergebnisse dieser Analyse steuert die Steuer- und Analyseeinheit 10 insbesondere die Stimulationseinheit 11 an. Zur Durchführung ihrer Aufgaben kann die Steuer- und Analyseeinheit 10 z.B. einen Prozessor (z.B. einen Mikrocontroller) enthalten.

Die Stimulationseinheit 11 kann z.B. ein Hirnschrittmacher sein und weist eine oder mehrere implantierbare Elektroden, z.B. Tiefenelektroden, sowie gegebenenfalls dazwischen geschaltete Verbindungskabel auf. Die Elektroden der Stimulationseinheit 11 bestehen typischerweise aus einem isolierten Elektrodenschaft und einer Mehrzahl von Stimulationskontakten (bzw. Stimulationskontaktflächen), die in den Elektrodenschaft eingebracht worden sind. In Fig. 1 sind beispielhaft vier Stimulationskontakte 25, 26, 27 und 28 dargestellt, die Stimulationseinheit 11 kann selbstverständlich aber auch eine andere Zahl von Stimulationskontakten aufweisen. Der Elektrodenschaft und die Stimulationskontakte 25-28 sind aus einem biokompatiblen Material hergestellt. Ferner sind die Stimulationskontakte 25-28 elektrisch leitfähig, beispielsweise sind sie aus einem Metall gefertigt, und befinden sich nach der Implantation in direktem elektrischen Kontakt mit dem Nervengewebe. Vorzugsweise kann jeder der Stimulationskontakte 25-28 über eine eigene Zuleitung angesteuert werden. Alternativ können auch zwei oder mehr Stimulationskontakte 25-28 an dieselbe Zuleitung angeschlossen sein. Die Stimulationskontakte 25-28 können beliebige Geometrien aufweisen, beispielsweise können sie rund oder rechteckig sein oder können sich zirkulär um den Elektrodenschaft herum erstrecken, und können ferner beliebig zueinander angeordnet sein.

Neben den Stimulationskontakten 25-28 kann jede Elektrode eine Referenzelektrode aufweisen, deren Oberfläche größer als die der Stimulationskontakte 25-28 sein kann. Z.B. kann auch das Generatorgehäuse als Referenz verwandt werden. Die Referenzelektrode wird bei der Stimulation des Nervengewebes zur Erzeugung eines Referenzpotentials eingesetzt. Alternativ kann auch einer der Stimulationskontakte 25-28 zu diesem Zweck verwendet werden. D.h., es kann entweder unipolar zwischen einem einzelnen Stimulationskontakt 25-28 und der Referenzelektrode oder bipolar zwischen verschiedenen Stimulationskontakten 25-28 stimuliert werden.

Die Messeinheit 12 enthält ein oder mehrere Sensoren, die es insbesondere ermöglichen, (i) eine Stimulus-induzierte Zurücksetzung der Phase der pathologischen oszillatorischen Aktivität und (ii) eine Ab- bzw. Zunahme der Amplitude der pathologischen oszillatorischen Aktivität nachzuweisen.

Als Sensoren können nicht-invasive Sensoren eingesetzt werden, z.B. Elektroenzephalographie (EEG)-Elektroden, Magnetenzephalographie (MEG)-Sensoren und Sensoren zur Messung lokaler Feldpotentiale (LFP). Die neuronale Aktivität kann auch indirekt durch Messung der damit einhergehenden Muskelaktivität mittels Elektromyographie (EMG) ermittelt werden.

Alternativ können die Sensoren in den Körper des Patienten implantiert sein. Als invasive Sensoren können beispielsweise epikortikale Elektroden, Tiefenhirnelektroden zur Messung von z.B. lokalen Feldpotentialen, sub- oder epidurale Hirnelektroden, subkutane EEG-Elektroden und sub- oder epidurale Rückenmarkselektroden dienen. Die Tiefenelektroden zur Messung der lokalen Feldpotentiale können auch baulich vereint oder sogar identisch mit den für die Stimulation verwendeten Tiefenelektroden sein.

Es kann durchaus vorgesehen sein, dass die einzelnen Komponenten der Vorrichtung 1, insbesondere die Steuer- und Analyseeinheit 10, die Stimulationseinheit 11 und/oder die Messeinheit 12, baulich voneinander getrennt sind. Die Vorrichtung 1 kann daher auch als System aufgefasst werden.

Die Vorrichtung 1 kann insbesondere zur Behandlung von neurologischen oder psychiatrischen Erkrankungen eingesetzt werden, z.B. Morbus Parkinson, essentiellem Tremor, Tremor infolge von Multipler Sklerose sowie anderen pathologischen Tremores, Dystonie, Epilepsie, Depression, Bewegungsstörungen, Kleinhirnerkrankungen, Zwangserkrankungen, Demenzerkrankungen, Morbus Alzheimer, Tourette-Syndrom, Autismus, Funktionsstörungen nach Schlaganfall, Spastik, Tinnitus, Schlafstörungen, Schizophrenie, Reizdarm-Syndrom, Suchterkrankungen, Borderline-Persönlichkeitsstörung, Aufmerksamkeits-Defizit-Syndrom, Aufmerksamkeits-Defizit-Hyperaktivitäts-Syndrom, Spielsucht, Neurosen, Fresssucht, Magersucht, Essstörungen, Burnout-Syndrom, Fibromyalgie, Migräne, Cluster-Kopfschmerz, allgemeiner Kopfschmerz, Neuralgie, Ataxie, Tic-Störung oder Hypertonie, sowie weiteren Erkrankungen, die durch krankhaft gesteigerte neuronale Synchronisation gekennzeichnet sind.

Die vorstehend genannten Krankheiten können durch eine Störung der bioelektrischen Kommunikation von Neuronenverbänden, die in spezifischen Schaltkreisen zusammengeschlossen sind, verursacht werden. Hierbei generiert eine Neuronenpopulation anhaltend krankhafte neuronale Aktivität und möglicherweise eine damit verbundene krankhafte Konnektivität (Netzwerkstruktur). Dabei bilden eine große Anzahl von Neuronen synchron Aktionspotentiale aus, d.h., die beteiligten Neuronen feuern übermäßig synchron. Hinzu kommt, dass die kranke Neuronenpopulation eine oszillatorische neuronale Aktivität aufweist, d.h., die Neuronen feuern rhythmisch. Im Fall von neurologischen oder psychiatrischen Erkrankungen liegt die mittlere Frequenz der krankhaften rhythmischen Aktivität der betroffenen Neuronenverbände etwa im Bereich von 1 bis 50 Hz, kann aber auch außerhalb dieses Bereichs liegen. Bei gesunden Menschen feuern die Neuronen hingegen qualitativ anders, z.B. auf unkorrelierte Weise.

In Fig. 1 ist die Vorrichtung 1 während einer CR-Stimulation dargestellt. Im Gehirn 29 oder Rückenmark 29 des Patienten weist mindestens eine Neuronenpopulation 30 eine wie vorstehend beschriebene krankhaft synchrone und oszillatorische neuronale Aktivität auf. Die Stimulationseinheit 11 stimuliert die krankhaft aktive Neuronenpopulation 30 im Gehirn 29 und/oder Rückenmark 29 mit den elektrischen Reizen 22 entweder direkt oder aber die Reize 22 werden über das Nervensystem an die krankhaft aktive Neuronenpopulation 30 weitergeleitet. Die elektrischen Reize 22 sind so ausgestaltet, dass die krankhaft synchrone Aktivität der Neuronenpopulation 30 desynchronisiert wird. Eine durch die Stimulation bewirkte Senkung der Koinzidenzrate der Neuronen kann zu einer Senkung der synaptischen Gewichte und somit zu einem Verlernen der Tendenz zur Produktion krankhaft synchroner Aktivität führen.

Die bei der CR-Stimulation verabreichten elektrischen Reize 22 bewirken in der Neuronenpopulation 30 ein Zurücksetzen, einen sogenannten Reset, der Phase der neuronalen Aktivität der stimulierten Neuronen. Durch das Zurücksetzen wird die Phase der stimulierten Neuronen unabhängig von dem aktuellen Phasenwert auf einen oder nahe zu einem bestimmten Phasenwert, z.B. 0°, gesetzt (in der Praxis ist es nicht möglich, einen bestimmten Phasenwert exakt einzustellen, dies ist für eine erfolgreiche CR-Stimulation aber auch nicht erforderlich). Somit wird die Phase der neuronalen Aktivität der krankhaften Neuronenpopulation 30 mittels einer gezielten Stimulation kontrolliert. Da die krankhafte Neuronenpopulation 30 über die Stimulationskontakte 25-28 an unterschiedlichen Stellen stimuliert wird, kann die Phase der neuronalen Aktivität der krankhaften Neuronenpopulation 30 an den unterschiedlichen Stimulationsstellen zu unterschiedlichen Zeitpunkten zurückgesetzt werden. Im Ergebnis wird dadurch die krankhafte Neuronenpopulation 30, deren Neuronen zuvor synchron und mit gleicher Frequenz und Phase aktiv waren, in mehrere Subpopulationen aufgespalten, die in Fig. 1 schematisch dargestellt sind und mit den Bezugszeichen 31, 32, 33 und 34 gekennzeichnet sind. Beispielsweise stimuliert der Stimulationskontakt 25 die Subpopulation 31, der Stimulationskontakt 26 stimuliert die Subpopulation 32, der Stimulationskontakt 27 stimuliert die Subpopulation 33 und der Stimulationskontakt 28 stimuliert die Subpopulation 34. Innerhalb jeder der Subpopulationen 31-34 sind die Neuronen nach dem Zurücksetzen der Phase weiterhin synchron und feuern auch weiterhin mit derselben pathologischen Frequenz, aber jede der Subpopulationen 31-34 weist bezüglich ihrer neuronalen Aktivität die Phase auf, die ihr durch den von dem jeweiligen Stimulationskontakt 25-28 generierten Stimulationsreiz aufgezwungen wurde. Dies bedeutet, dass die neuronalen Aktivitäten der einzelnen Subpopulationen 31-34 nach dem Zurücksetzen ihrer Phasen weiterhin einen in etwa sinusförmigen Verlauf mit derselben pathologischen Frequenz haben, aber unterschiedliche Phasen.

Bedingt durch die krankhafte Interaktion zwischen den Neuronen ist der durch die Stimulation erzeugte Zustand mit mindestens zwei Subpopulationen instabil, und die gesamte Neuronenpopulation 30 nähert sich schnell einem Zustand kompletter Desynchronisation, in welchem die Neuronen unkorreliert feuern. Der gewünschte Zustand, d.h. die komplette Desynchronisation, ist somit nach der zeitversetzten (oder phasenverschobenen) Applikation der phasenrücksetzenden Reize 22 nicht sofort vorhanden, sondern stellt sich meist innerhalb weniger Perioden oder gar in weniger als einer Periode der pathologischen Frequenz ein.

Eine Theorie zur Erklärung des Stimulationserfolgs basiert darauf, dass die letztlich gewünschte Desynchronisation durch die krankhaft gesteigerte Interaktion zwischen den Neuronen erst ermöglicht wird. Hierbei wird ein Selbstorganisationsprozess ausgenutzt, der für die krankhafte Synchronisation verantwortlich ist. Derselbe bewirkt, dass auf eine Aufteilung einer Gesamtpopulation 30 in Subpopulationen 31-34 mit unterschiedlichen Phasen eine Desynchronisation folgt. Im Gegensatz dazu würde ohne krankhaft gesteigerte Interaktion der Neuronen keine Desynchronisation erfolgen.

Darüber hinaus kann durch die CR-Stimulation eine Neuorganisation der Konnektivität der gestörten neuronalen Netzwerke erzielt werden, so dass lang anhaltende therapeutische Effekte bewirkt werden können. Der erzielte synaptische Umbau ist von großer Bedeutung für die wirksame Behandlung neurologischer oder psychiatrischer Erkrankungen.

Im Folgenden wird die Eichung beschrieben, die mit der Vorrichtung 1 durchgeführt wird, um damit die optimalen Stimulationsorte und insbesondere die optimalen Stimulationsparameter für die elektrische CR-Stimulation zu ermitteln.

Ein wesentlicher Aspekt der Eichung besteht darin, aus einer Gruppe von vorhandenen Stimulationskontakten diejenigen auszusuchen, welche nach der Implantation in das Gehirn oder Rückenmark des Patienten in der Lage sind, eine Phasenrücksetzung der pathologischen Oszillation zu bewirken. Die pathologische Oszillation wird z.B. mittels (des Makrosignals) EEG gemessen; sie entspricht auf der Mikroebene einer krankhaft übersteigerten Synchronisation der einzelnen Neuronen innerhalb der betroffenen Neuronenpopulation(en). Die Stimulationskontakte, über welche jeweils eine Phasenrücksetzung bewirkt werden kann, werden weiter dadurch getestet, dass sie paarweise als CR-Stimulation angewandt nicht zu einem Anwachsen der Synchronisation (d.h. der Amplitude der pathologischen Oszillation führen) und dass über die aus lauter derartigen wirksamen Paaren von Stimulationskontakten bestehende Gesamtgruppe von N (> 2) Stimulationskontakten zu einer effektiven CR-Stimulation verwendet werden kann, d.h., dass diese CR-Stimulation desynchronisierend wirkt, so dass die Amplitude der pathologischen Oszillation sinkt. Falls N = 2, wird nur die desynchronisierende Wirkung des Paares von Stimulationskontakten untersucht.

Es kann auch vorgesehen sein, dass der Schritt mit der Austestung der Paare von Stimulationskontakten weggelassen wird und gleich mit der Testung der desynchronisierenden Wirkung der N Stimulationskontakte fortgefahren wird.

Das vorstehend skizzierte Vorgehen kann je nach medizinischen Anforderungen unterschiedlich ausgestaltet werden. Zur Veranschaulichung werden im Folgenden zwei Varianten beschrieben.

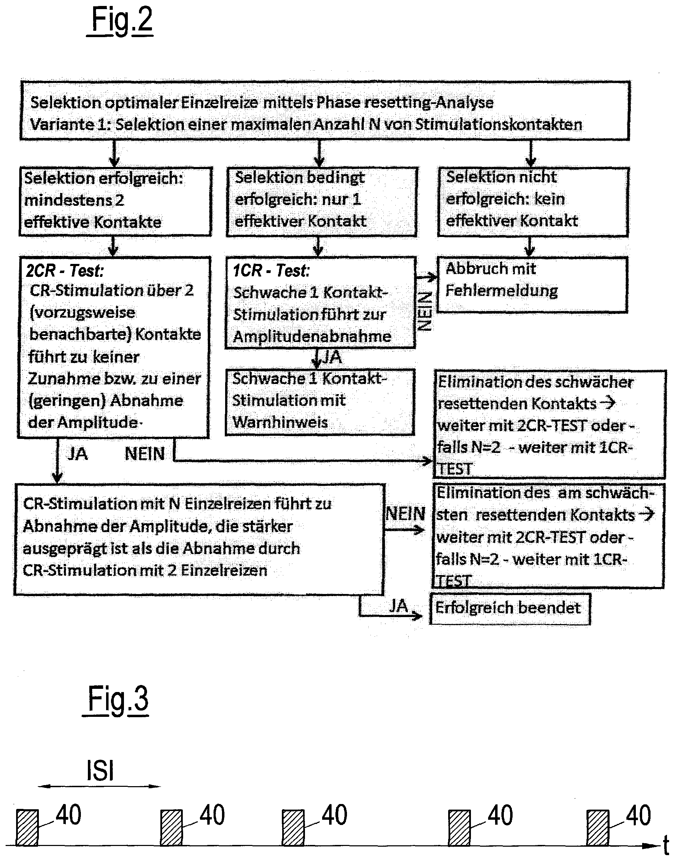

Die gemäß einer ersten Variante durchgeführten Schritte sind im Flussdiagram von Fig. 2 zusammengefasst. Diese Variante wird dann gewählt, wenn möglichst viele wirksame (d.h. die Phase der pathologischen Oszillation zurücksetzende) Stimulationskontakte ausgewählt werden sollen. Aus allen vorhandenen Stimulationskontakten werden die N Stimulationskontakte ausgewählt, die eine Phasenrücksetzung der pathologischen Oszillation der Hirnaktivität (bzw. der Muskelaktivität) bewirken. Es kann auch eine Untergruppe aller (physikalisch) vorhandenen Stimulationskontakte verwendet werden. Z.B. können das obere, mittlere oder untere (an der Elektrodenspitze liegende) Drittel aller Stimulationskontakte einer Elektrode ausgewählt werden. Auch können alle Stimulationskontakte ausgewählt werden, die - gemäß Zusatzinformationen - besonders aussichtsreich sind. Solche Zusatzinformationen sind z.B.: Aus Kernspinuntersuchungen und/oder Computertomographieuntersuchungen rekonstruierte Informationen bzgl. der Lage der Elektrode (samt Stimulationskontakten) in Relation zur Ausdehnung des zu stimulierenden Zielgebiets oder einer zu stimulierenden Faserbahn. Zusätzlich können noch darauf aufbauende numerische Simulationen von Computermodellen als Hinweis für optimale Stimulationskontakte verwendet werden, um die zu testenden Stimulationskontakte entsprechend a priori einzuschränken. Solche Zusatzinformation kann aber auch nur zum Vergleich mit der funktionalen Austestung herangezogen werden. Entsprechen z.B. die Ergebnisse der funktionellen Austestung den Ergebnissen der anatomischen Rekonstruktion und dynamischen Computersimulation, so kann von der höchsten Verlässlichkeit der Kalibration ausgegangen werden.

Aus allen vorhandenen Stimulationskontakten bzw. einer oben beschriebenen Untergruppe derselben wird zunächst die größtmögliche Anzahl N von Stimulationskontakten selektiert, welche die Phase der pathologischen oszillatorischen Hirnaktivität (bzw. der Muskelaktivität) zu resetten vermögen. Dazu werden alle Stimulationskontakte bzw. die Stimulationskontakte einer Untergruppe einzeln ausgetestet, indem in einem ersten Schritt elektrische Reize über jeden dieser Stimulationskontakte an das Nervengewebe verabreicht werden. Die Reizstärke wird dabei so gewählt, dass die Reize in der Lage sind, eine Phasenrücksetzung der pathologischen Oszillation (welche einer synchronen neuronalen Aktivität entspricht) zu bewirken. Falls die anfängliche Reizstärke zu schwach gewählt wurde, wird die Reizstärke über eine entsprechende Einstellung der Reizparameter erhöht. Zu den einstellbaren Reizparametern zählen die Amplitude der Einzelpulse, die Dauer der Einzelpulse, die Pulsfrequenz innerhalb des Pulszugs und die Anzahl der Einzelpulse im Pulszug. Die Austestung der Reizparameter erfolgt mittels einer Analyse der Phasenrücksetzung, z.B. der weiter unten beschriebenen "phase resetting"-Analyse.

Die Analyse der Phasenrücksetzüng der pathologischen Oszillation erfolgt typischerweise mittels eines Ensembles von identischen Reizen. Beispielhaft ist in Fig. 3 ein solches Ensemble von elektrischen Reizen 40 gegen die Zeit t aufgetragen, die nacheinander von jedem der Stimulationskontakte appliziert werden. Zur Vermeidung von "Entrainment"-Phänomenen sollte ein Interstimulus-Intervall ISI zwischen den einzelnen Reizen 40 von hinreichend großer und randomisierter Länge eingehalten werden. Das mittlere Interstimulus-Intervall sollte im Vergleich zur eigentlichen Reizantwort lang genug sein, damit die Reizantworten nicht überlappen und bei Verabreichung des nachfolgenden Reizes 40 vollständig abgeklungen sind.

Bei den Reizen 40 handelt es sich vorzugsweise um Bursts, d.h. um kurze Hochfrequenz-Pulszüge (wobei die einzelnen Pulse ladungsbalanciert sind). Die Parameter der Reize 40 hängen stark von der verwendeten Elektrodengeometrie und den Stimulationskontaktflächen ab und dürfen dem Fachmann bekannte Sicherheits-Grenzwerte nicht überschreiten, damit es zu keinen gewebeschädigenden Effekten kommt (vgl. "Selection of stimulus parameters for deep brain stimulation" von A. M. Kuncel und W. M. Grill (erschienen in Clin. Neurophysiol. 115, 2004, Seiten 2431 bis 2441) und "Matching geometry and stimulation parameters of electrodes for deep brain stimulation experiments - numerical considerations" von U. Gimsa et al. (erschienen in J. Neurosci. Methods 150, 2006, Seiten 212 bis 227)). Bei bestimmten derzeit für den klinischen Gebrauch verwendeten Abmessungen der Stimulationskontaktflächen liegt die Amplitude der Einzelpulse z.B. im Bereich von 0.2 mA bis 4 mA und in seltenen Fällen bis 6 mA. Die Dauer der Einzelpulse beträgt zwischen 10 µs und 500 µs und insbesondere zwischen 60 µs und 200 µs. In einem Pulszug befinden sich zwischen 1 und 20 und insbesondere zwischen 3 und 9 Einzelpulse. Die Frequenz innerhalb eines Pulszugs beträgt zwischen 80 Hz und 500 Hz, bevorzugt zwischen 100 Hz und 200 Hz, z.B. 130 Hz.

Anhand der von der Messeinheit in Reaktion auf die Applikation der Reize 40 aufgenommenen Messsignale ermittelt die Steuer- und Analyseeinheit, ob die von den einzelnen Stimulationskontakten applizierten Reize 40 die Phase der pathologischen, synchronisierten und oszillatorischen Hirnaktivität zurücksetzen. Dabei wählt die Steuer- und Analyseeinheit von den getesteten Stimulationskontakten in einem zweiten Schritt diejenigen Stimulationskontakte aus, welche die Phase der pathologischen, synchronisierten und oszillatorischen Hirnaktivität (bzw. der Muskelaktivität) zurückzusetzen vermögen. Verfahren zur Überprüfung einer derartigen Phasenrücksetzung sind dem Fachmann bekannt.

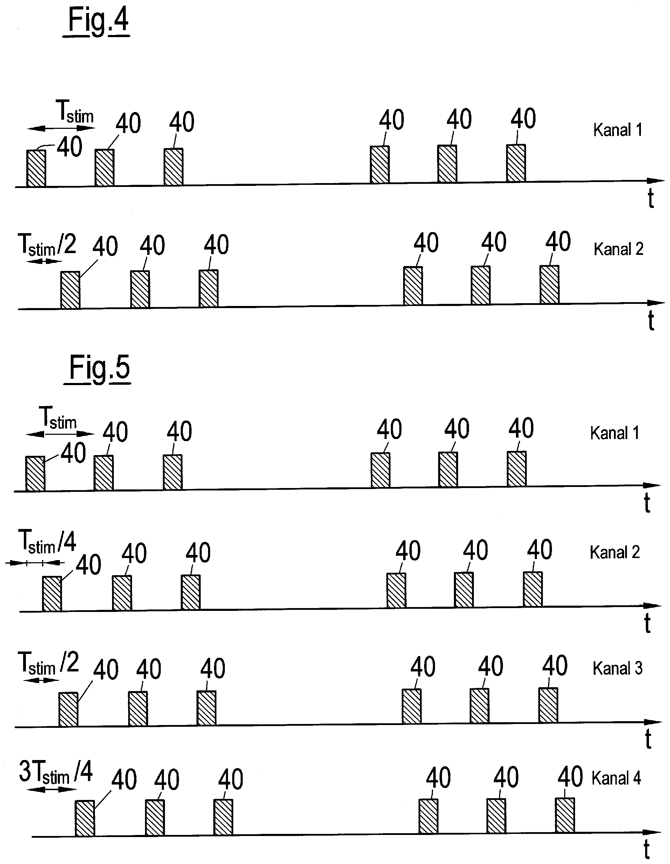

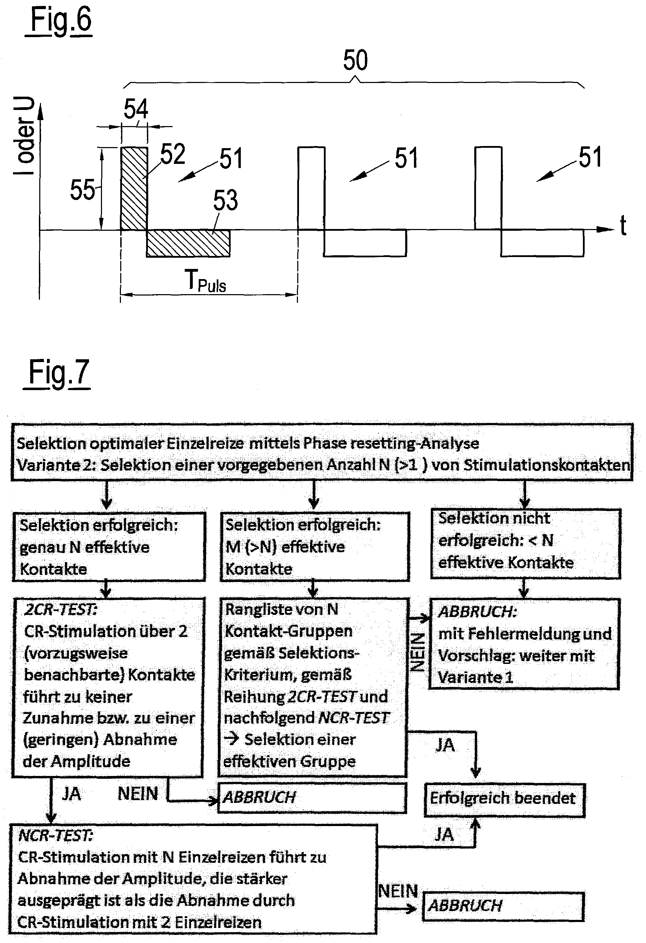

Eine Möglichkeit, die dem Fachmann zur Analyse der Phasenrücksetzung geläufig ist, besteht aus einer "phase resetting"-Analyse mittels einzeln applizierter elektrischer Reize (bestehend aus einem Einzelreiz oder aus einem hochfrequenten Pulspaket mit intra-burst-Frequenz von vorzugsweise > 100 Hz, z.B. 130 Hz, wie weiter unten im Zusammenhang mit Fig. 6 beschrieben wird), wie sie beispielsweise in dem Artikel "Stochastic phase resetting of two coupled phase oscillators stimulated at different times" von P. A. Tass (erschienen in Physical Review E 67, 2003, Seiten 051902-1 bis 051902-15) beschrieben ist. Dazu wird der "phase resetting"-Index ermittelt (vgl. Gleichung 8, "stimulus-locking index" für ν = 1). Die hierbei zur Berechnung der Phasenrücksetzung verwandte Phase wird z.B. mittels der Hilbert-Transformation aus dem mittels Bandpassfilterung bzw. "empirical mode decomposition" bestimmten Signal, welches die pathologische oszillatorische Aktivität repräsentiert, ermittelt (letztere ermöglicht im Vergleich zur Bandpassfilterung eine parameterunabhängige Bestimmung physiologisch relevanter Moden in verschiedenen Frequenzbereichen, vgl. "The empirical mode decomposition and the Hilbert spectrum for nonlinear and non-stationary time series analysis" von N. E. Huang et al. (erschienen in Proceedings of the Royal Society of London Series A, 1998, Band 454, Seiten 903 bis 995); die Kombination "empirical mode decomposition" mit nachfolgender Hilbert-Transformation wird als Hilbert-Huang-Transformation bezeichnet, vgl. "A confidence limit for the empirical mode decomposition and Hilbert spectral analysis" von N. E. Huang et al. (erschienen in Proceedings of the Royal Society of London Series A, 2003, Band 459, Seiten 2317 bis 2345)). Eine Phasenrücksetzung ist erreicht, wenn der "phase resetting"-Index die 99. Perzentile der prä-Stimulus-Verteilung des "phase resetting"-Index überschreitet (vgl. Fig. 4 in "Stochastic phase resetting of two coupled phase oscillators stimulated at different times" von P. A. Tass). Falls medizinisch eine stärkere phasenrücksetzende Wirkung wünschenswert ist, können auch höhere Schwellen, z.B. das Doppelte oder das Dreifache der 99. Perzentile der prä-Stimulus-Verteilung des "phase resetting"-Index gewählt werden.

Alternativ zu dieser Datenanalyse können auch einfachere Datenanalyse-Verfahren angewandt werden, die die Detektion der Phasenrücksetzung mit in der Praxis hinreichender Genauigkeit zu approximieren vermögen. Z.B. kann einfach über das Ensemble an Reizantworten gemittelt werden. Von einer Phasenrücksetzung ist dann approximativ auszugehen, wenn der maximale Betrag der Reizantwort die 99. Perzentile der prä-Stimulus-Verteilung der gemittelten Antwort (oder deren Doppeltes bzw. Dreifaches) überschreitet (vgl. Fig. 6 in "Stochastic phase resetting of two coupled phase oscillators stimulated at different times" von P. A. Tass).

Falls kein Hirnsignal (LFP, EEG, MEG, durch Inversverfahren aus EEG- und/oder MEG-Signalen berechnete zeitabhängige Hirnströme bzw. Dipolmomente), sondern die Muskelaktivität im Falle eines Patienten mit pathologischem Tremor (Zittern) verwendet wird, ist eine andere (dem Fachmann bekannte) Vorverarbeitung des Signals der Muskelaktivität (z.B. dem Oberflächen-EMG) durchzuführen: Zuerst wird das Oberflächen-EMG hochpass-gefiltert (z.B. > 25 Hz), um nur die Burst-Aktivität zu extrahieren, aber Bewegungsartefakte (im Frequenzbereich des pathologischen Tremors, z.B. bei 5 Hz) zu eliminieren. Als nächster Schritt wird das hochpass-gefilterte Signal rektifiziert, d.h., es wird der Betrag genommen. Dieses Signal wird dann wie ein Hirnsignal (LFP, EEG, MEG, durch Inversverfahren aus EEG- und/oder MEG-Signalen berechnete zeitabhängige Hirnströme bzw. Dipolmomente) weiterverarbeitet.

Die EEG- und MEG-Signale werden z.B. entweder nach entsprechender Vorverarbeitung (z.B. dem Fachmann geläufige Elimination von Artefakten wie Blinzelartefakte) direkt oder aber nach Ermittlung der zugrunde liegenden Hirnströme mittels dem Fachmann bekannten Verfahren für die Rückwärtsrechnung (mittels räumlich verteilten Stromdichten oder mehreren Dipolen) analysiert. In letzterem Fall wird der Zeitgang von Hirnströmen bzw. Dipolmomenten analysiert. Dies ermöglicht, eine spezifisch auf die phasenrücksetzende Wirkung in einem oder mehreren besonders relevanten Hirnarealen abgestimmte Eichung durchzuführen.

In einem dritten Schritt wird getestet, ob die Stimulationskontakte im Vergleich zur Ausdehnung der zu stimulierenden Neuronenpopulation hinreichend weit voneinander entfernt sind (um zu vermeiden, dass zwei oder mehr Stimulationskontakte dieselbe Subpopulation stimulieren). Hierzu wird über benachbarte Stimulationskontakte jeweils eine 2 Kontakt-CR-Stimulation (2CR-TEST) durchgeführt. Fig. 4 zeigt einen solchen 2CR-TEST mit zwei benachbarten Stimulationskontakten, die jeweils einem Kanal entsprechen. Über jeden der beiden Stimulationskontakte werden die elektrischen Reize 40 in einer Sequenz periodisch mit der Periode Tₛₜᵢₘ appliziert. In dem vorliegenden Fall umfasst jede Sequenz drei elektrische Reize 40, die Sequenzen können allerdings auch weitere Reize 40 enthalten. Nach jeder Sequenz wird eine Pause eingehalten und die Sequenz danach wiederholt. Die Pause zwischen den Sequenzen kann z.B. ein ganzzahliges Vielfaches der Periode Tₛₜᵢₘ betragen. Ferner beträgt die zeitliche Verzögerung zwischen den Sequenzen unterschiedlicher Kanäle Tₛₜᵢₘ/2, wobei von diesem Wert um z.B. bis zu ± 5%, ± 10% oder ± 20% abgewichen werden kann.

Die Periode Tₛₜᵢₘ wird nahe bei der mittleren Periode der pathologischen Oszillation gewählt. Beispielsweise wird die Stimulationsfrequenz fₛₜᵢₘ = 1/Tₛₜᵢₘ beim 2CR-TEST (und ebenso beim weiter unten beschriebenen NCR-TEST) entweder angepasst an das zu desynchronisierende Frequenzband gewählt (z.B. bei pathologischer Synchronisation im Deltaband eine darin bzw. noch besser in der unteren Hälfte befindliche Stimulationsfrequenz, also z.B. 1,5 Hz) oder - z.B. vor Beginn jeder Testung quasi online - an den Peak im Powerspektrum des pathologischen Frequenzbands angepasst. In letzterem Fall wird die Stimulationsfrequenz fₛₜᵢₘ so gewählt, dass diese 1:1 der Peak-Frequenz oder - weniger bevorzugt - einem kleineren n:m-Vielfachen derselben entspricht (n, m sind ganze Zahlen und vorzugsweise < 10). Ferner kann ein Literaturwert für die mittlere Periode der pathologischen Oszillation herangezogen werden, und die für die Stimulation verwendete Periode Tₛₜᵢₘ kann von diesem Literaturwert um z.B. bis zu ± 5%, ± 10% oder ± 20% abweichen. Typischerweise liegt die Stimulationsfrequenz fₛₜᵢₘ im Bereich von 1 bis 35 Hz.

Von der Steuer- und Analyseeinheit werden die in Reaktion auf den 2CR-TEST aufgenommenen Messsignale analysiert. Die Amplitude der pathologischen Oszillation sollte durch die in Fig. 4 gezeigte Stimulation nicht erhöht werden, im günstigen Fall kann sogar eine leichte Abnahme (entspricht einer schwachen Desynchronisation) auftreten. Im Vergleich zur gesamten Ausdehnung der Neuronenpopulation zu dicht liegende Stimulationskontakte können im ungünstigen Fall zu einer Zunahme der Synchronisation, d.h. zu einem Anwachsen der Amplitude der pathologischen Oszillation, führen. Dies sollte vermieden werden. In diesem Falle wird dementsprechend aus solch einem Paar von Stimulationskontakten, welches im 2CR-TEST synchronisierend wirkt, der Stimulationskontakt eliminiert, der gemäß der Eingangsuntersuchung eine schwächer phasenrücksetzende Wirkung hat. Der 2CR-TEST wird für alle Nachbarpaare aus den zuvor ausgewählten N Stimulationskontakten mit gegebenenfalls nachfolgender Elimination ungeeigneter Stimulationskontakte durchgeführt und kann so häufig angewandt werden, bis schließlich ein Gruppe von N' (≤ N) Stimulationskontakten übrig bleibt.

In einem vierten Schritt wird eine CR-Stimulation über all diese N' Stimulationskontakte durchgeführt (NCR-TEST), wie sie z.B. in Fig. 5 für N' = 4 gezeigt ist. In jedem der vier Kanäle (jeder Kanal entspricht einem wirksamen Stimulationskontakt) wird der elektrische Reiz 40 in einer Sequenz periodisch mit der Periode Tₛₜᵢₘ appliziert, wobei Tₛₜᵢₘ auch hier wie oben beschrieben nahe bei der mittleren Periode der pathologischen Oszillation liegt (typischerweise liegt fₛₜᵢₘ = 1/Tₛₜᵢₘ im Bereich von 1 bis 35 Hz). In dem vorliegenden Beispiel umfasst jede Sequenz drei Reize 40, die Sequenzen können allerdings auch mehr Reize 40 enthalten. Nach jeder Sequenz wird eine bestimmte Pause eingehalten und die Sequenz danach wiederholt. Ferner beträgt die zeitliche Verzögerung zwischen den Sequenzen benachbarter Kanäle Tₛₜᵢₘ/4, da vier Kanäle vorhanden sind. Für den allgemeinen Fall von N' Kanälen würde die zeitliche Verzögerung benachbarter Kanäle Tₛₜᵢₘ/N' betragen (von diesem Wert kann auch um z.B. bis zu ± 5%, ± 10% oder ± 20% abgewichen werden).

Wenn diese CR-Stimulation die krankhaft synchrone und oszillatorische neuronale Aktivität der stimulierten Neuronen unterdrückt und insbesondere desynchronisierend wirkt (d.h. wenn die Amplitude der pathologischen Oszillation signifikant unter die Amplitude vor Stimulationsbeginn fällt; entsprechende Signifikanztests sind dem Fachmann bekannt), werden diese N' Stimulationskontakte zur Behandlung des Patienten ausgewählt. Sollte dies nicht der Fall sein, muss ein weniger effizienter Stimulationskontakt eliminiert werden; und die Prozedur beginnt beim 2CR-TEST von neuem bzw. geht beim 1CR-TEST weiter.

Der 1CR-TEST wird im suboptimalen Fall verwendet, wenn nur ein Stimulationskontakt wirksam ist (d.h. eine Phasenrücksetzung bewirkt). In diesem Fall wird ausgetestet, ob eine schwache (d.h. im Vergleich zur Standard-Hochfrequenzstimulation mit einer um den Faktor 2 oder - besser - 3 geringeren Amplitude durchgeführte) ein Kontakt-CR-Stimulation, d.h. eine periodische Applikation von Stimulationsbursts, eine signifikante Verminderung der Amplitude der pathologischen Oszillation bewirkt. Sollte dies der Fall sein, kann von der Steuer- und Analyseeinheit diese Variante als Behandlungsmodus gewählt werden. Die Steuer- und Analyseeinheit sollte aber auf jeden Fall einen Warnhinweis liefern, in dem deutlich gemacht wird, dass die ein Kontakt-CR-Stimulation deutlich suboptimal ist: Sie wirkt deutlich langsamer als eine Mehrkanal-CR-Stimulation und birgt bei hohen Stimulationsstärken die Gefahr der synchronisierenden Wirkung.

Sollte im Rahmen der Testprozedur nicht mal ein einziger wirksamer Stimulationskontakt übrig bleiben, so liefert die Steuer- und Analyseeinheit eine entsprechende Fehlermeldung und es kommt zum Abbruch des Tests.

Die erste Variante kann - wie schon beschrieben - auch mit der Randbedingung vorgenommen werden, dass die maximale Anzahl von effektiven Stimulationskontakten aus einer Untermenge von Stimulationskontakten selektiert werden soll. Die Untermenge kann z.B. primär vorrichtungsorientiert definiert sein (z.B. das obere oder mittlere oder unter Drittel aller Stimulationskontakte) oder durch zusätzliche z.B. anatomische Informationen (Stimulationskontakte, die gemäß Bildgebung im Zielgebiet liegen).

Fig. 6 zeigt beispielhaft einen Pulszug 50, der als Einzelreiz 40 in den in den Fig. 3, 4 und 5 gezeigten Stimulationsverfahren eingesetzt werden kann. Der Pulszug 50 kann aus 1 bis 100, insbesondere 2 bis 10, elektrischen ladungsbalancierten Einzelpulsen 51 bestehen (in Fig. 6 sind beispielhaft 3 Einzelpulse 51 gezeigt). Die Einzelpulse 51 werden innerhalb des Pulszugs 50 mit einer Frequenz f_{Puls} = 1/T_{Puls} im Bereich von 50 bis 500 Hz, insbesondere im Bereich von 100 bis 150 Hz, wiederholt. Die Einzelpulse 51 können strom- oder spannungskontrollierte Pulse sein, die sich aus einem anfänglichen Pulsanteil 52 und einem sich daran anschließenden, in entgegengesetzter Richtung fließenden Pulsanteil 53 zusammensetzen, wobei die Polarität der beiden Pulsanteile 52 und 53 gegenüber der in Fig. 6 gezeigten Polarität auch vertauscht werden kann. Außerdem kann eine Pause einer Länge von bis zu 20 ms zwischen beide Pulsanteile 52, 53 eingefügt werden. Die Dauer 54 des Pulsanteils 52 liegt im Bereich zwischen 1 µs und 500 µs. Die Amplitude 55 des Pulsanteils 52 liegt im Falle von stromkontrollierten Pulsen im Bereich zwischen 0 mA und 25 mA und im Fall von spannungskontrollierten Pulsen im Bereich von 0 bis 20 V. Die Amplitude des Pulsanteils 53 ist geringer als die Amplitude 55 des Pulsanteils 52. Dafür ist die Dauer des Pulsanteils 53 länger als die des Pulsanteils 52. Die Pulsanteile 52 und 53 sind idealerweise so dimensioniert, dass die Ladung, welche durch sie übertragen wird, bei beiden Pulsanteilen 52 und 53 gleich groß ist, d.h. die in Fig. 6 schraffiert eingezeichneten Flächen sind gleich groß. Demnach wird durch einen Einzelpuls 51 genauso viel Ladung in das Gewebe eingebracht, wie aus dem Gewebe entnommen wird.

Die in Fig. 6 dargestellte Rechteckform der Einzelpulse 51 stellt eine ideale Form dar. Je nach der Güte der die Einzelpulse 51 erzeugenden Elektronik wird von der idealen Rechteckform abgewichen.

Anstelle von elektrischen Einzelpulsen oder Pulszügen können auch anders ausgestaltete elektrische Reize bei der CR-Stimulation eingesetzt werden, z.B. zeitlich kontinuierliche Reizmuster. Die oben beschriebenen Signalformen und deren Parameter sind daher nur beispielhaft zu verstehen. Es kann durchaus vorgesehen sein, dass von den oben angegebenen Signalformen und deren Parametern abgewichen wird.

Im Folgenden wird eine zweite Eich-Variante zur Bestimmung der optimalen Reizparameter und Stimulationsorte für die elektrische CR-Stimulation beschrieben, deren wesentliche Schritte im Flussdiagram von Fig. 7 zusammengefasst sind.

Bei entsprechender Elektrodengeometrie, z.B. bei Vorliegen von sehr vielen Stimulationskontakten, kann es medizinisch vorteilhaft sein, die CR-Stimulation nicht über möglichst viele, sondern über eine optimale Anzahl von Stimulationskontakten durchzuführen. In diesem Fall wird zu Beginn der Eichung die gewünschte Anzahl N von Stimulationskontakten gewählt. Sollten genau N Stimulationskontakte eine Phasenrücksetzung der pathologischen Oszillation bewirken, wird (wie bei der ersten Variante) in einem 2CR-TEST und nachfolgenden NCR-TEST untersucht, ob die CR-Stimulation, über alle N Stimulationskontakte appliziert, zu einer signifikanten Abnahme der Amplitude der pathologischen Oszillation führt.

Sollten M (> N) Stimulationskontakte eine Phasenrücksetzung der pathologischen Oszillation bewirken, wird gemäß eines Selektionskriteriums eine Rangliste möglicher N-Kontakt-Gruppen erstellt.

Gemäß einem ersten Selektionskriterium werden aus den M Stimulationskontakten die N am weitesten voneinander entfernten Stimulationskontakte ausgewählt. Sollte es zwei oder mehr Möglichkeiten geben, werden diejenigen Stimulationskontakte mit dem größten kumulativen "phase resetting"-Index (Maximalwert summiert über alle Stimulationskontakte) ausgewählt.

Gemäß einem zweiten Selektionskriterium werden aus M Stimulationskontakten diejenigen N Stimulationskontakte ausgewählt, welche die stärkste Phasenrücksetzung bewirken.

Gemäß einem dritten Selektionskriterium werden aus M Stimulationskontakten diejenigen N Stimulationskontakte ausgewählt, welche den größten Überlapp haben mit der durch eine anatomische Rekonstruktion (und gegebenenfalls zusätzlicher dynamischer Computersimulation) ermittelten Randbedingung (des zu stimulierenden Zielgebiets). Es können z.B. diejenigen N Stimulationskontakte ausgewählt werden, die dem Zielgebiet am nächsten liegen.

Gemäß dieser Rangliste wird (analog zum oben beschriebenen Vorgehen) mittels 2CR-TEST und nachfolgendem NCR-TEST die optimale Gruppe mit N Stimulationskontakten ermittelt. Sollte dies unmöglich sein, wird entweder eine ein Kontakt-CR-Stimulation oder aber keine CR-Stimulation als bestmögliche Therapie selektiert.

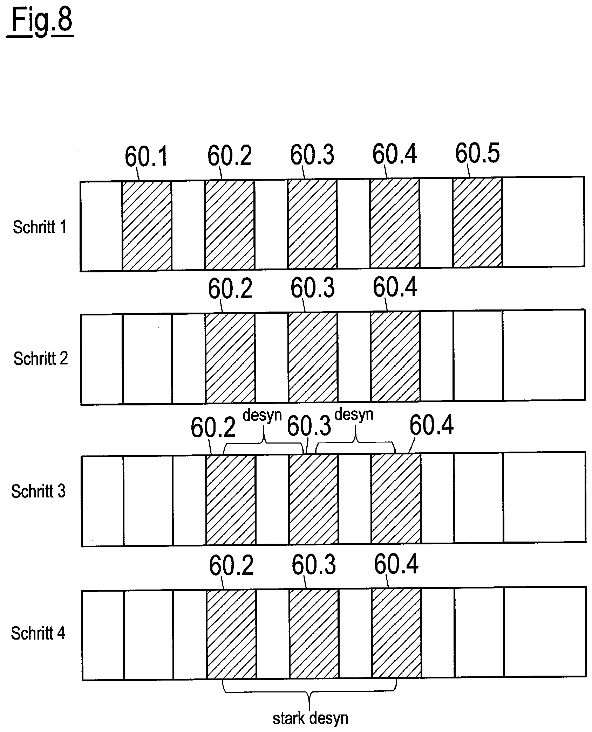

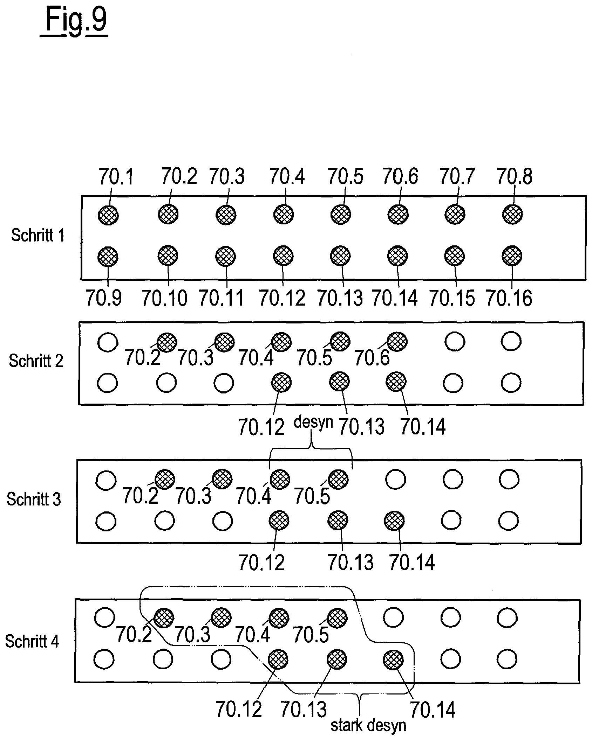

Fig. 8 und 9 veranschaulichen die Kalibrationsprozedur gemäß der oben beschriebenen ersten Variante an zwei unterschiedlichen Elektrodengeometrien.

Die in Fig. 8 schematisch gezeigte Elektrode weist fünf zirkuläre Stimulationskontakte 60.1-60.5 auf. Links in der Darstellung von Fig. 8 ist die Elektrodenspitze (nicht detaillierter skizziert). Die horizontale Dimension zeigt die Längsausrichtung der Elektrode. Die Außenfläche (d.h. die Kontaktfläche mit dem Nervengewebe) der Elektrode ist aufgeschnitten (d.h. obere und untere Längskante waren vor dem Aufschneiden verschmolzen.).

Im Schritt 1 werden über jeden der Stimulationskontakte 60.1-60.5 elektrische Reize an das Nervengewebe verabreicht. Anhand der dabei aufgenommenen Messsignale, z.B. EEG- und/oder MEG-Signalen, ermittelt die Steuer- und Analyseeinheit im Schritt 2 die Stimulationskontakte, welche eine wirksame Phasenrücksetzung der pathologischen Oszillation bewirken. Im Beispiel von Fig. 8 erfüllen die Stimulationskontakte 60.2-60.4 dieses Kriterium. Mit diesen drei Stimulationskontakten wird im Schritt 3 der 2CR-TEST durchgeführt. Die beim 2CR-TEST vorgenommenen CR-Stimulationen mit jeweils zwei benachbarten Stimulationskontakten, d.h. CR-Stimulationen mit den Paaren 60.2/60.3 und 60.3/60.4, führt zu keiner Erhöhung der Amplitude der pathologischen Oszillation. Daher wird anschließend im Schritt 4 mit allen drei Stimulationskontakten 60.2-60.4 der NCR-TEST durchgeführt. Die im Rahmen dieses Tests vorgenommene CR-Stimulation mit den drei Stimulationskontakten 60.2-60.4 wirkt stark desynchronisierend auf die stimulierte Neuronenpopulation. Als Ergebnis liefert die Kalibrationsprozedur demnach die Stimulationskontakte 60.2-60.4, mit denen sich optimale Ergebnisse bei der nachfolgenden CR-Therapie erzielen lassen.

Fig. 9 zeigt schematisch eine Elektrode mit 16 kreisförmigen Stimulationskontakten 70.1-70.16, wobei in der Darstellung von Fig. 9 links die Elektrodenspitze ist (nicht detaillierter skizziert). Die horizontale Dimension zeigt die Längsausrichtung der Elektrode. Die Außenfläche (d.h. die Kontaktfläche mit dem Nervengewebe) der Elektrode ist aufgeschnitten. D.h., die obere und die untere Längskante waren vor dem Aufschneiden verschmolzen.

Im Schritt 1 wird auf alle vorhandenen Stimulationskontakte 70.1-70.16 (und keine Untergruppe davon) die Kalibrationsprozedur angewandt. Im Schritt 2 ermittelt die Steuer- und Analyseeinheit mittels der während des Schritts 1 aufgenommenen EEG- und/oder MEG-Signalen die Stimulationskontakte, die eine wirksame Phasenrücksetzung der pathologischen Oszillation bewirken. Im vorliegenden Beispiel sind dies die Stimulationskontakte 70.2-70.6 und 70.12-70-14. Im Schritt 3 wird mit allen benachbarten Paaren der im Schritt 2 ausgewählten Stimulationskontakte der 2CR-TEST durchgeführt. Dabei werden horizontale Nachbarpaare (z.B. 70.3/70.4), vertikale Nachbarpaare (z.B. 70.4/70.12) und diagonale Nachbarpaare (z.B. 70.3/70.12) ausgetestet. In dem vorliegenden Ausführungsbeispiel wird hierbei der Stimulationskontakt 70.6 eliminiert. Anschließend wird im Schritt 4 der NCR-TEST mit den verbleibenden Stimulationskontakten 70.2-70.5 und 70.12-70.14 durchgeführt und es zeigt sich, dass eine CR-Stimulation mit diesen Stimulationskontakten zu einer starken Desynchronisation der stimulierten Neuronenpopulation führt.

Durch derartige objektive, systematisch durchzuführende Untersuchungen wird die klinische Austestung (z.B. die Beobachtung der Auswirkung der Stimulation auf die Kardinalsymptome des Parkinson, also auf Tremor, Rigor und Akinese) ersetzt. Dadurch werden subjektive und zum Teil begrenzt verlässliche Eindrücke des evaluierenden Arztes durch eine elektrophysiologisch basierte Messung der Reizantworten des Gehirns zur Eichung der optimalen Stimulationsparameter und Stimulationsorte ersetzt.

Für die nachfolgende Therapie können unterschiedliche Arten der CR-Stimulation verwandt werden. Eine Möglichkeit besteht in einer "N aus N"-CR-Stimulation, d.h. pro Stimulationszyklus Tₛₜᵢₘ werden wie in Fig. 5 (für N = 4) von allen N ausgewählten Stimulationskontakten Einzelreize 40 appliziert. Alternativ kann auch eine "L aus N"-CR-Stimulation (mit L < N) durchgeführt werden, bei der pro Stimulationszyklus Tₛₜᵢₘ aus N Stimulationskontakten L z.B. randomisiert selektiert werden und von diesen die Reize 40 appliziert werden. Auf diese Weise kann eine größere räumliche Variabilität erzeugt werden.

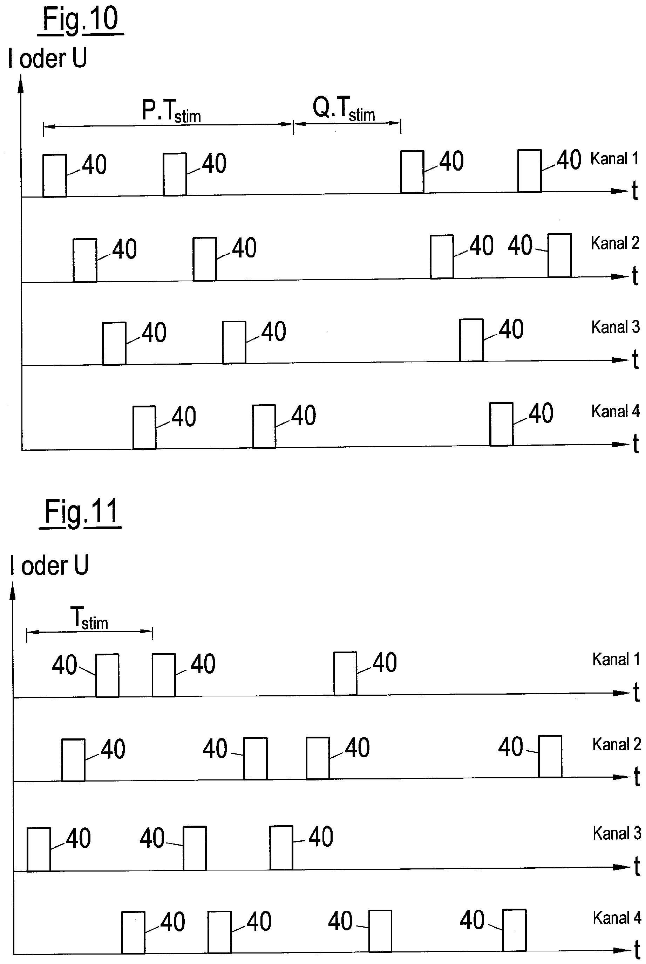

Weitere Variationen der CR-Stimulation mit vier mittels der Kalibrationsprozedur bestimmten Stimulationskontakten (N = 4) sind in den Fig. 10 und 11 gezeigt.

Fig. 10 zeigt eine Pause, die während der Applikation der Reize 40 vorgesehen werden kann und während der keine Stimulation erfolgt. Derartige Pausen können beliebig lang gewählt werden und insbesondere ein ganzzahliges Vielfaches der Stimulationsperiode Tₛₜᵢₘ betragen. Ferner können die Pausen nach einer beliebigen Anzahl von Stimulationen eingehalten werden. Z.B. kann eine Stimulation während P aufeinander folgender Perioden der Länge Tₛₜᵢₘ durchgeführt werden und anschließend eine Pause während Q Perioden der Länge Tₛₜᵢₘ ohne Stimulation eingehalten werden, wobei P und Q kleine ganze Zahlen sind, z.B. im Bereich von 1 bis 20. Dieses Schema kann entweder periodisch fortgesetzt werden oder stochastisch und/oder deterministisch, z.B. chaotisch, modifiziert werden.

Eine weitere Möglichkeit, von dem in Fig. 5 gezeigten streng periodischen Stimulationsmuster abzuweichen, besteht darin, die zeitliche Abfolge der Reize 40 stochastisch oder deterministisch oder gemischt stochastischdeterministisch zu variieren. Fig. 11 zeigt, dass die Reihenfolge, in welcher die einzelnen Stimulationskontakte die Reize 40 applizieren, pro Periode Tₛₜᵢₘ (oder auch in anderen Zeitschritten) variiert wird. Diese Variation kann stochastisch oder deterministisch oder gemischt stochastischdeterministisch erfolgen.

Die in Fig. 11 gezeigte Randomisierung kann mit der in Fig. 10 gezeigten Stimulationsform kombiniert werden. Beispielsweise kann in jedem der P aufeinander folgenden Stimulationszeitabschnitte der Länge Tₛₜᵢₘ eine erneute Randomisierung durchgeführt werden oder aber es erfolgt nach jeder Pause der Länge Q x Tₛₜᵢₘ eine Randomisierung und innerhalb der darauf folgenden P Stimulationszeitabschnitte bleibt die Reihenfolge, in welcher die Stimulationskontakte die Reize 40 applizieren, konstant.

Des Weiteren kann von dem in Fig. 5 gezeigten streng periodischen Stimulationsmuster abgewichen werden, indem die zeitliche Verzögerung zwischen zwei aufeinander folgenden Reizen 40 nicht stets gleich groß ist. Es kann vorgesehen sein, dass die zeitlichen Abstände zwischen den Reizen 40 unterschiedlich gewählt werden. Ferner können die Verzögerungszeiten auch während der Behandlung eines Patienten variiert werden. Auch können die Verzögerungszeiten hinsichtlich der physiologischen Signallaufzeiten adjustiert werden.

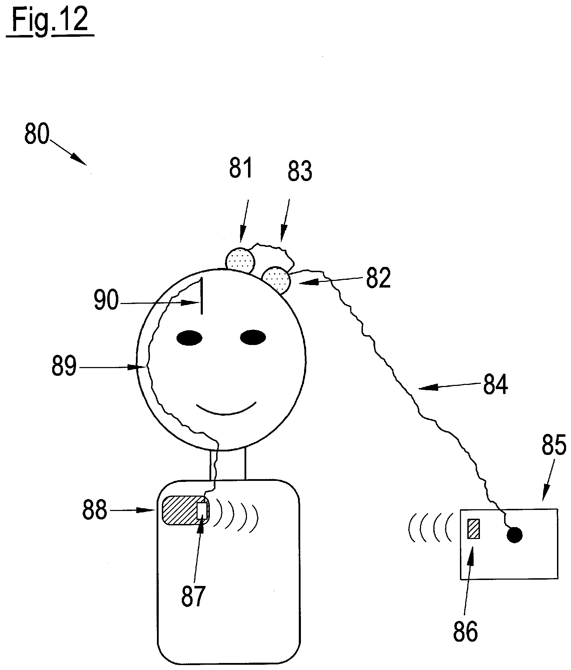

Fig. 12 zeigt schematisch eine Vorrichtung 80 zur EEG-basierten Eichung der CR-Stimulation, appliziert über implantierte Hirnelektroden (z.B. Tiefenelektroden), für die Behandlung von neurologischen und psychiatrischen Erkrankungen mit pathologisch gesteigerter neuronaler Synchronisation, z.B. Parkinson, Dystonie, pathologischer Tremores (z.B. essentieller Tremor), Epilepsien, Gilles de la Tourette-Syndrom, Zwangserkrankungen, Depressionen, Alzheimer, Demenz, schweren Suchterkrankungen und schwersten Persönlichkeitsstörungen. Nicht-invasiv fixierte EEG-Elektroden 81, 82 messen die EEG-Reizantworten und übermitteln die jeweiligen EEG-Reizantworten über Kabel 83, 84 an die zentrale Steuer-, Verstärker- und Analyseeinheit 85. Letztere kommuniziert bidirektional über eine entsprechende Sende- und Empfangseinheit 86 telemetrisch mit der telemetrischen Sende- und Empfangseinheit 87 des implantierten Generators 88. Letzterer ist über ein ableitendes Kabel 89 mit einer oder mehreren implantierten Tiefenelektroden 90 verbunden. Elektrische Testreize werden über die Tiefenelektrode 90 verabreicht. Die hierzu verwendeten Steuersignale werden vom Generator 88 generiert - gemäß den telemetrisch übermittelten Vorgaben von der Steuer-, Verstärker- und Analyseeinheit 85. Letztere führt die Datenanalyse der EEG-Reizantworten durch.

Anstelle eines implantierten Generators und einer mit dem Generator telemetrisch kommunizierenden, nicht-implantierten Steuer-, Verstärker- und Analyseeinheit kann die Steuer-, Verstärker- und Analyseeinheit auch in das Generatorgehäuse integriert sein. Die Steuer-, Verstärker- und Analyseeinheit kann in diesem Fall ein standardisiertes Programm von Testreizen ablaufen lassen, und aus den EEG-Artefakten (approximativ) die Stimulations-Zeitpunkte ermitteln. In einer anderen Variante ist der Generator über ein ausgeleitetes Kabel direkt mit der implantierten Tiefenelektrode verbunden. Der Nachteil hierbei ist, dass eine derartige Ausleitung ein Infektionsrisiko in sich birgt, so dass die Ausleitung gemäß gegenwärtiger Praxis (gemäß entsprechender Studienergebnisse) in vielen klinischen Zentren nur in den ersten 10 Tagen nach Elektrodenimplantation durchgeführt wird. In dieser Zeit liegt bei einem Teil der Patienten infolge der Implantation der Makroelektrode aber noch ein Ödem im vorderen Bereich der Elektrode vor (welches im weiteren Verlauf abklingt), so dass die Neuronenpopulation dort sowohl ein verändertes Spontanverhalten (z.B. deutlich weniger oder sogar gar keine krankhaft synchrone Aktivität) als auch veränderte Reizantworten aufweisen kann.

## Patentansprüche

1. Vorrichtung (1) zur Stimulation von Neuronen mit einer krankhaft synchronen und oszillatorischen neuronalen Aktivität, umfassend
- eine Stimulationseinheit (11) mit einer Mehrzahl von Stimulationskontakten (25-28) zur Stimulation von Neuronen im Gehirn und/oder Rückenmark eines Patienten mit elektrischen Reizen (22),
- eine Messeinheit (12) zum Aufnehmen von Messsignalen (23), die eine neuronale Aktivität der stimulierten Neuronen wiedergeben, und
- eine Steuer- und Analyseeinheit (10) zur Steuerung der Stimulationseinheit (11) und zur Analyse der Messsignale (23),
**dadurch gekennzeichnet, dass** die Steuer- und Analyseeinheit (10) derart ausgestaltet ist, dass sie
- die Stimulationseinheit (11) derart ansteuert, dass die Stimulationskontakte (25-28) Reize (22) applizieren,
- anhand der in Reaktion auf die Applikation der Reize (22) aufgenommenen Messsignale (23) die Stimulationskontakte (25-28) auswählt, deren Reize (22) eine Phasenrücksetzung der krankhaft synchronen und oszillatorischen neuronalen Aktivität der stimulierten Neuronen bewirken,
- die Stimulationseinheit (11) derart ansteuert, dass die ausgewählten Stimulationskontakte (25-28) phasenrücksetzende Reize (22) zeitversetzt applizieren, und
- anhand der in Reaktion auf die zeitversetzt applizierten phasenrücksetzenden Reize (22) aufgenommenen Messsignale (23) prüft, ob die zeitversetzt applizierten phasenrücksetzenden Reize (22) die krankhaft synchrone und oszillatorische neuronale Aktivität der stimulierten Neuronen unterdrücken.

2. Vorrichtung (1) nach Anspruch 1, wobei die Steuer- und Analyseeinheit (10) derart ausgestaltet ist, dass sie
- die Stimulationseinheit (11) derart ansteuert, dass die ausgewählten Stimulatioriskontakte (25-28) mit den zeitversetzt applizierten phasenrücksetzenden Reizen (22) eine "Coordinated Reset"-Stimulation durchführen.

3. Vorrichtung (1) nach Anspruch 1 oder 2, wobei die Steuer- und Analyseeinheit (10) derart ausgestaltet ist, dass sie
- die Stimulationseinheit (11) derart ansteuert, dass die ausgewählten Stimulationskontakte (25-28) paarweise phasenrücksetzende Reize (22) zeitversetzt applizieren, und
- anhand der in Reaktion auf die von einem Paar von Stimulationskontakten (25-28) zeitversetzt applizierten phasenrücksetzende Reize (22) aufgenommenen Messsignale (23) prüft, ob die von dem Paar von Stimulationskontakten (25-28) zeitversetzt applizierten phasenrücksetzenden Reize (22) einen Anstieg der krankhaft synchronen und oszillatorischen Aktivität der Neuronen bewirken.

4. Vorrichtung (1) nach Anspruch 3, wobei die Steuer- und Analyseeinheit (10) derart ausgestaltet ist, dass sie,
- falls die von dem Paar von Stimulationskontakten (25-28) zeitversetzt applizierten phasenrücksetzenden Reize (22) einen Anstieg der krankhaft synchronen und oszillatorischen Aktivität der Neuronen bewirken, einen der beiden Stimulationskontakte (25-28) verwirft.

5. Vorrichtung (1) nach Anspruch 4, wobei die Steuer- und Analyseeinheit (10) derart ausgestaltet ist, dass sie
- denjenigen Stimulationskontakt (25-28) verwirft, der mittels der phasenrücksetzenden Reize (22) eine schwächere Phasenrücksetzung der neuronalen Aktivität der stimulierten Neuronen bewirkt.

6. Vorrichtung (1) nach einem der Ansprüche 3 bis 5, wobei die Stimulationskontakte eines Paars von Stimulationskontakten (25-28) benachbart zueinander angeordnet sind.

7. Vorrichtung (1) nach einem der vorhergehenden Ansprüche, wobei die Steuer- und Analyseeinheit (10) derart ausgestaltet ist, dass sie aus einer Anzahl M der Stimulationskontakte (25-28), mit denen sich eine Phasenrücksetzung der krankhaft synchronen und oszillatorischen neuronalen Aktivität der stimulierten Neuronen erzielen lässt, diejenigen N Stimulationskontakte (25-28) auswählt,
- die am weitesten voneinander entfernt liegen, und/oder
- die mittels der phasenrücksetzenden Reize (22) die stärkste Phasenrücksetzung der neuronalen Aktivität der stimulierten Neuronen bewirken, und/oder
- die den größten Überlapp mit dem Zielgebiet im Gehirn und/oder Rückenmark des Patienten haben.

8. Vorrichtung (1) nach einem der vorhergehenden Ansprüche, wobei die Steuer- und Analyseeinheit (10) diejenigen Stimulationskontakte (25-28) auswählt, die bei einer "Coordinated Reset"-Stimulation die besten Ergebnisse liefern.

## Claims

1. An apparatus (1) for stimulating neurons with a pathologically synchronous and oscillatory neural activity, comprising
- a stimulation unit (11) having a plurality of stimulation contacts (25-28) for stimulating neurons in the brain and/or spinal cord of a patient using electrical stimuli (22);
- a measuring unit (12) for recording measured signals (23) which reproduce a neural activity of the stimulated neurons; and
- a control and analysis unit (10) for controlling the stimulation unit (11) and for analyzing the measured signals (23),
**characterized in that** the control and analysis unit (10) is configured such that it
- controls the stimulation unit (11) such that the stimulation contacts (25-28) apply stimuli (22);
- selects the stimulation contacts (25-28), whose stimuli (22) effect a phase reset of the pathologically synchronous and oscillatory neural activity of the stimulated neurons, with reference to the measured signals (23) recorded in response to the application of the stimuli (22);
- controls the stimulation unit (11) such that the selected stimulation contacts (25-28) apply phase resetting stimuli (22) with a time offset; and
- checks with respect to the measured signals (23) recorded in response to the phase resetting stimuli (22) applied with a time offset whether the phase resetting stimuli (22) applied with a time offset suppress the pathologically synchronous and oscillatory neural activity of the stimulated neurons.

2. An apparatus (1) in accordance with claim 1, wherein the control and analysis unit (10) is configured such that it
- controls the stimulation unit (11) such that the selected stimulation contacts (25-28) carry out a coordinated reset stimulation using the phase resetting stimuli (22) applied with a time offset.

3. An apparatus (1) in accordance with claim 1 or claim 2, wherein the control and analysis unit (10) is configured such that it
- controls the stimulation unit (11) such that the selected stimulation contacts (25-28) apply phase resetting stimuli (22) pairwise with a time offset; and
- checks with reference to the measured signals (23) recorded in response to the phase-resetting stimuli (22) applied with a time offset by a pair of stimulation contacts (25-28) whether the phase resetting stimuli (22) applied with a time offset by the pair of stimulation contacts (25-28) effect an increase in the pathologically synchronous and oscillatory activity of the neurons.

4. An apparatus (1) in accordance with claim 3, wherein the control and analysis unit (10) is configured such that it
- discards one of the two stimulation contacts (25-28) if the phase-resetting stimuli (22) applied with a time offset by the pair of stimulation contacts (25-28) effect an increase in the pathologically synchronous and oscillatory activity of the neurons.

5. An apparatus (1) in accordance with claim 4, wherein the control and analysis unit (10) is configured such that it
- discards that stimulation contact (25-28) which effects a weaker phase reset of the neural activity of the stimulated neurons by means of the phase resetting stimuli (22).

6. An apparatus (1) in accordance with any one of the claims 3 to 5, wherein the stimulation contacts of a pair of stimulation contacts (25-28) are arranged adjacent to one another.

7. An apparatus (1) in accordance with any one of the preceding claims, wherein the control and analysis unit (10) is configured such that it selects those N stimulation contacts (25-28) from a number M of the stimulation contacts (25-28) with which a phase reset of the pathologically synchronous and oscillatory neural activity of the stimulated neurons can be achieved
- which lie furthest remote from one another; and/or
- which effect the greatest phase reset of the neural activity of the stimulated neurons by means of the phase resetting stimuli (22); and/or
- which have the greatest overlap with the target zone in the brain and/or spinal cord of the patient.

8. An apparatus (1) in accordance with any one of the preceding claims, wherein the control and analysis unit (10) selects those stimulation contacts (25-28) which deliver the best results in a coordinated reset stimulation.

## Revendications

1. Dispositif (1) pour la stimulation de neurones présentant une activité pathologique synchrone et oscillatoire, incluant
- une unité de stimulation (11) avec une pluralité de contacts de stimulation (25-28) pour la stimulation de neurones dans le cerveau et/ou dans la moelle épinière d'un patient avec des excitations électriques (22),
- une unité de mesure (12) pour enregistrer des signaux de mesure (23) qui reproduisent une activité neuronale des neurones stimulés, et
- une unité de commande et d'analyse (10) pour commander l'unité de stimulation (11) et pour analyser les signaux de mesure (23),
**caractérisé en ce que** l'unité de commande et d'analyse (10) est conçue de telle façon que :
- elle pilote l'unité de stimulation (11) de telle façon que les contacts de stimulation (25-28) appliquent des excitations (22),
- au moyen des signaux de mesure (23) enregistrés en réaction à l'application des excitations (22), elle sélectionne les contacts de stimulation (25-28) dont les excitations (22) provoquent une remise en phase de l'activité neuronale pathologique synchrone et oscillatoire des neurones stimulés,
- elle pilote l'unité de stimulation (11) de telle façon que les contacts de stimulation sélectionnés (25-28) appliquent des excitations de remise en phase (22) de façon décalée dans le temps, et
- au moyen des signaux de mesure (23) enregistrés en réaction aux excitations (22) de remise en phase appliquées de façon décalée dans le temps, elle vérifie si les excitations (22) de remise en phase appliquées de façon décalée dans le temps suppriment l'activité neuronale pathologique synchrone et oscillatoire des neurones stimulés.

2. Dispositif (1) selon la revendication 1, dans lequel l'unité de commande et d'analyse (10) est conçue de telle façon que
- elle pilote l'unité de stimulation (11) de telle façon que les contacts de stimulation sélectionnés (25-28) exécutent, avec les excitations (22) de remise en phase appliquées de façon décalée dans le temps, une stimulation dite "Coordinated Reset" (à remise à zéro coordonnée).

3. Dispositif (1) selon la revendication 1 ou 2, dans lequel l'unité de commande et d'analyse (10) est conçue de telle façon que
- elle pilote l'unité de stimulation (11) de telle façon que les contacts de stimulation sélectionnés (25-28) appliquent des excitations de remise en phase (22) paire par paire et de façon décalée dans le temps, et
- au moyen des signaux de mesure (23) enregistrés en réaction aux excitations de remise en phase (22) appliquées de façon décalée dans le temps par une paire de contacts de stimulation (25-28), elle vérifie si les excitations de remise en phase (22) appliquées de façon décalée dans le temps par la paire de contacts de stimulation (25-28) entraînent une augmentation de l'activité pathologique synchrone et oscillatoire des neurones.

4. Dispositif (1) selon la revendication 3, dans lequel l'unité de commande et d'analyse (10) est conçue de telle façon que
- dans le cas où les excitations de remise en phase (22) appliquées de façon décalée dans le temps par la paire de contacts de stimulation (25-28) entraînent une augmentation de l'activité pathologique synchrone et oscillatoire des neurones, elle rejette l'un des deux contacts de stimulation (25-28).

5. Dispositif (1) selon la revendication 4, dans lequel l'unité de commande et d'analyse (10) est conçue de telle façon que
- elle rejette celui des contacts de stimulation (25-28) qui, au moyen des excitations de remise en phase (22), entraîne une remise en phase plus faible de l'activité neuronale des neurones stimulés.

6. Dispositif (1) selon l'une des revendications 3 à 5, dans lequel les contacts de stimulation d'une paire de contacts de stimulation (25-28) sont agencée au voisinage l'un de l'autre.

7. Dispositif (1) selon l'une des revendications précédentes, dans lequel l'unité de commande et d'analyse (10) est conçue de telle façon que, à partir d'un nombre M des contacts de stimulation (25-28), au moyen desquels il est possible d'atteindre une remise en phase de l'activité neuronale pathologique synchrone et oscillatoire des neurones stimulés, elle sélectionne les N contacts de stimulation (25-28) qui
- sont éloignés les uns des autres à la plus grande distance et/ou
- entraînent, au moyen des excitations de remise en phase (22), la plus forte remise en phase de l'activité neuronale des neurones stimulés, et/ou
- présentent le plus grand chevauchement avec la zone cible dans le cerveau et/ou dans la moelle épinière du patient.

8. Dispositif (1) selon l'une des revendications précédentes, dans lequel l'unité de commande et d'analyse (10) sélectionne ceux des contacts de stimulation (25-28) qui, lors d'une stimulation du type "Coordinated Reset", fournissent les meilleurs résultats.
